# EUROPEAN PATENT APPLICATION

(11) **EP 3 081 166 A1**
(43) Date of publication of application: **19.10.2016**
(21) Application number: 13898899.3
(22) Date of filing: 09.12.2013
(51) Int. Cl.: A61B 8/00

(54) **METHOD AND DEVICE FOR ADJUSTING SETTING VALUES OF ULTRASOUND IMAGE**

(71) Applicant: Alpinion Medical Systems Co., Ltd., Hwaseong-si, Gyeonggi-do 445-380 (KR)
(72) Inventor: CHO, Hyunchul, Ansan-si Gyeonggi-do 426-749 (KR); KANG, Seung-pum, Yangju-si Gyeonggi-do 482-834 (KR); CHANG, Sun-yeob, Seoul 122-010 (KR); SON, Keonho, Seongnam-si Gyeonggi-do 463-440 (KR)
(74) Representative: Thorniley, Peter
(86) International application number: PCT/KR2013/011353
(87) International publication number: WO 2015/088054

(57) **Abstract**

A method and an apparatus for adjusting an ultrasound image setting are are provided. A method and an apparatus for adjusting an ultrasound image setting provide an interface configured to apply or adjust an image parameter related to an ultrasound image such that a plurality of image parameters (for example, a frame rate, an image quality, and so on) related to the ultrasound image can be simultaneously adjusted by only one operation (a single input signal).

## Description

### [Technical Field]

The present disclosure relates to a method and an apparatus for adjusting an ultrasound image setting.

### [Background Art]

The statements in this section merely provide background information related to the present disclosure and do not necessarily constitute prior art.

An ultrasound medical apparatus utilizes a probe for transmitting ultrasound to a subject, receiving a reflection signal from the subject, and converting the reflection signal to an electrical signal in order to generate an ultrasound image.

Such an ultrasound medical apparatus includes a user input unit configured to receive a control instruction of a user. The user can operate the user input unit to select an image mode according to the kind of a subject or the purpose of a diagnosis, and set an image parameter of an image mode. Here, this leaves a user who is supposed to check an ultrasound image with an added difficulty of meticulously setting a plurality of complicated image parameters according to certain subject and the particular diagnostic purpose.

### [Summary of Invention]

### [Technical Problem]

Therefore, the present disclosure provides a method and an apparatus for adjusting an ultrasonic image setting to provide an interface for application or adjustment of an image parameter related to an ultrasound image so as to enable a single operation or a single input signal to simultaneously adjust a plurality of image parameters, for example, a frame rate, an image quality, and so on related to the ultrasound image.

### [Solution to Problem]

In accordance with some embodiments of the present disclosure, an ultrasound medical apparatus includes a user input unit, a transmission control unit and a control unit. The user input unit is configured to receive a user instruction. The transmission control unit is configured to operate elements of a transducer for outputting a focused ultrasound or an unfocused ultrasound. The control unit is configured to be responsive to an input position determined on a first interface by the user instruction, for generating an input signal corresponding to the input position and causing one of the focused ultrasound and the unfocused ultrasound to be selected based on the input signal.

In accordance with another embodiment of the present disclosure, a method for adjusting an ultrasound image setting includes detecting whether an input position is determined on a first interface by a user instruction, generating an input signal corresponding to the determined input position, and controlling to cause one of a focused ultrasound and an unfocused ultrasound to be selected based on the input signal.

### [Advantageous Effects of Invention]

According to some embodiments of the present disclosure as described above, a plurality of image parameters related to an ultrasound image can be simultaneously adjusted with only one operation by providing an interface for application or adjustment of the image parameters.

In addition, at least one embodiment of the present disclosure obviates the need for discriminating complicated image parameters for an ultrasound image setting to obtain a desired ultrasound image.

### [Brief Description of Drawings]

FIG. 1 is a schematic block diagram of an ultrasound medical apparatus according to at least one embodiment of the present disclosure.
FIG. 2A is a diagram of an ultrasound image setting menu according to at least one embodiment of the present disclosure.
FIG. 2B is a diagram of an ultrasound image setting menu according to another embodiment of the present disclosure.
FIG. 3 is a flowchart of a method ffor adjusting an ultrasound image setting according to at least one embodiment of the present disclosure.
FIG. 4 is a diagram of a display parameter adjustment according to at least one embodiment of the present disclosure.
FIG. 5 is a diagram of a method for combining some of a plurality of image parameters according to at least one embodiment of the present disclosure.

### [Description of Embodiments]

Hereinafter, at least one embodiment of the present disclosure will be described in detail with reference to the accompanying drawings.

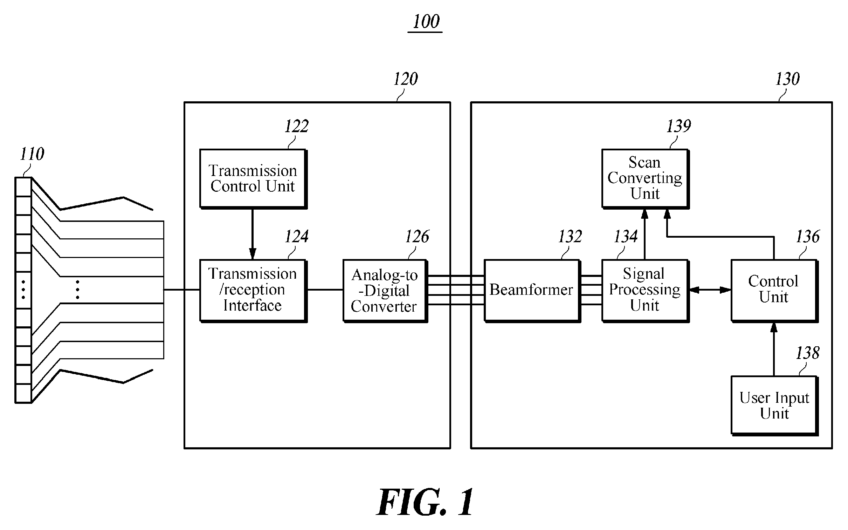

FIG. 1 is a schematic block diagram of an ultrasound medical apparatus 100 according to at least one embodiment of the present disclosure.

The ultrasound medical apparatus 100 performs a software-based beamforming, and includes a transducer 110, a front processing unit or front end 120 and a host 130. The ultrasound medical apparatus 100 according to some embodiments of the present disclosure is not necessarily limited to this configuration.

The front end 120 may include a transmission control unit 122, a transmission/reception interface 124 and an analog-to-digital converter 126. In addition, the host 130 may include a beamformer 132, a signal processing unit 134, a control unit 136, a user input unit 138 and a scan converting unit 139. The host 130 may perform a software-based parallel processing for high-speed image processing, and its architectural equivalent may have a multi-core CPU (Central Processing Unit) and a GPU (Graphic Processing Unit) for carrying out the parallel processing in thousands of channels at the same time.

The front end 120 and the host 130 can be connected via a full parallel path in order to perform the software-based high-speed image processing, and may use, for example, a PCI express (Peripheral Component Interconnect Express) interface therefor.

The ultrasound medical apparatus 100 according to the embodiment performs the software-based high-speed image processing, and therefore an image processing control such as the control of a frame rate, an image quality, an image size, and so on, can be easily performed thanks to a connecting structure of the full parallel path between the front end 120 and the host 130. Accordingly, an interface is provided for allowing a user to select an image mode according to the kind of a subject or the purpose of a diagnosis and then more easily perform a software-based adjustment of an image parameter of each image mode. The interface enables the user to more easily control functions related to the high-speed image processing with the ultrasound medical apparatus 100.

The transducer 110 converts an electrical analog signal into an ultrasound (focused ultrasound or unfocused ultrasound) to transmit the converted ultrasound to a subject, and converts a signal reflected from the subject (hereinafter, referred to as a reflected signal) into an electrical analog signal. In addition, the transducer 110 may be implemented as a transducer array.

The transducer 110 according to some embodiments can transmit the focused ultrasound to the subject according to instructions of the control unit 136 delivered via the transmission control unit 122 and receive the reflected signal corresponding to the focused ultrasound from the subject, or the transducer 110 can transmit the unfocused ultrasound (for example, a plane wave) from the subject according to the instructions of the control unit 136 delivered via the transmission control unit 122 and receive the reflected signal corresponding to the unfocused ultrasound from the subject. Here, the reflected signal can be processed through the software-based high-speed image processing.

Hereinafter, components included in the front end 120 will be described.

The transmission control unit 122 applies a voltage pulse to the transducer 110 via the transmission/reception interface 124, and causes the focused ultrasound or the unfocused ultrasound to be output from transducer elements of the transducer 110. The transmission/reception interface 124 has a function of switching between transmission and reception such that the transducer 110 alternately performs the transmission and the reception. The analog-to-digital converter 126 converts the reflected analog signal received from the transmission/reception interface 124 into a digital signal and then transmits the digital signal to the beamformer 132.

Hereinafter, components included in the host 130 will be described.

The beamformer 132 delays an electrical signal appropriate for the transducer 110 to convert the delayed electrical signal into an electrical signal matched to each of the transducer elements. In addition, the beamformer 132 delays or adds up the electrical signals converted by the respective transducer elements to calculate the electrical signal as an output value of the corresponding transducer element. The beamformer 132 includes a transmission beamformer, a reception beamformer and a beamforming unit. Meanwhile, the beamformer 132 may be connected to the analog-to-digital converter 126 and the signal processing unit 134 via the full parallel path in order to perform the software-based high-speed image processing.

The signal processing unit 134 converts the reflected signals on received scanlines focused by the beamformer 132 into baseband signals, and detects an envelope to obtain data of one scan line by using a quadrature demodulator. In addition, the signal processing unit 134 processes the data generated by the front end 120 into digital signals.

The signal processing unit 134 performs the software-based parallel processing for the high-speed image processing, and its architecture equivalent may have a multi-core CPU and a GPU for carrying out the parallel processing in thousands of channels at the same time.

The control unit 136 according to some embodiments is a control means configured to control the entire operation related to application or adjustment of the image parameters of the ultrasound image. The control unit 136 also controls the transmission control unit 122 as well as performs a control instruction according to commands by the user input unit 138. For example, the control unit 136 may control the transmission control unit 122 to select the focused ultrasound or the unfocused ultrasound according to the commands by the user input unit 138.

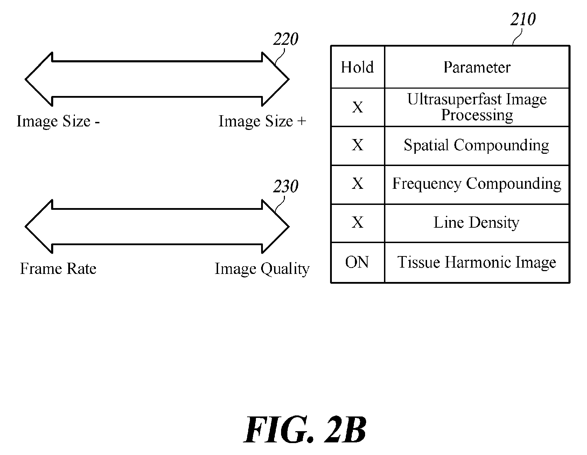

The control unit 136 is responsive to a control instruction (user instruction) for the ultrasound image setting received from the user input unit 138 for controlling the signal processing unit 134 to display a first interface 200 configured to apply or adjust a plurality of (at least two) image parameters of the ultrasound image on a display installed in the ultrasound medical apparatus 100. Here, as shown in FIG. 2B, the first interface 200 in display may be divided into a third interface 220 and a fourth interface 230.

When the input position on the first interface 200 is determined by the user instructions of the user input unit 138, the control unit 136 generates an input signal corresponding to the input position and controls the transmission control unit 122 based on the input signal to select any one of the focused ultrasound and the unfocused ultrasound. In addition, the control unit 136 combines some of the plurality of image parameters and applies the combined image parameters to the ultrasound image based on the input position on the first interface 200. Here, the first interface 200 includes a focus area and an unfocus area for adjusting an ultrasound image. Based on the input position in the focus area, the control unit 136 combines the focusing parameters (focused mode of parameters among the plurality of image parameters) to apply the combined focusing parameters to the ultrasound image. Based on the input position in the unfocus area, the control unit 136 combines the unfocusing parameters (unfocused mode of parameters among the plurality of image parameters) to apply the combined unfocusing parameters to the ultrasound image.

The control unit 136 is configured to cause the plurality of image parameters to be applied or cause setting values corresponding to the plurality of image parameters to be adjusted based on the input position on the first interface 200. The control unit 136 is configured to set some parameters (e.g., frame-rate parameters and image-quality related parameters) among the plurality of image parameters in a trade-off relation based on the input position. Here, basically, the plurality of image parameters includes frame rate parameters and image-quality related parameters as requisite by default, and may include image-size related parameters as optional.

In addition, the control unit 136 controls the transmission control unit 122 to select any one of the focused ultrasound and the unfocused ultrasound based on the input position of the input signal on the first interface 200.

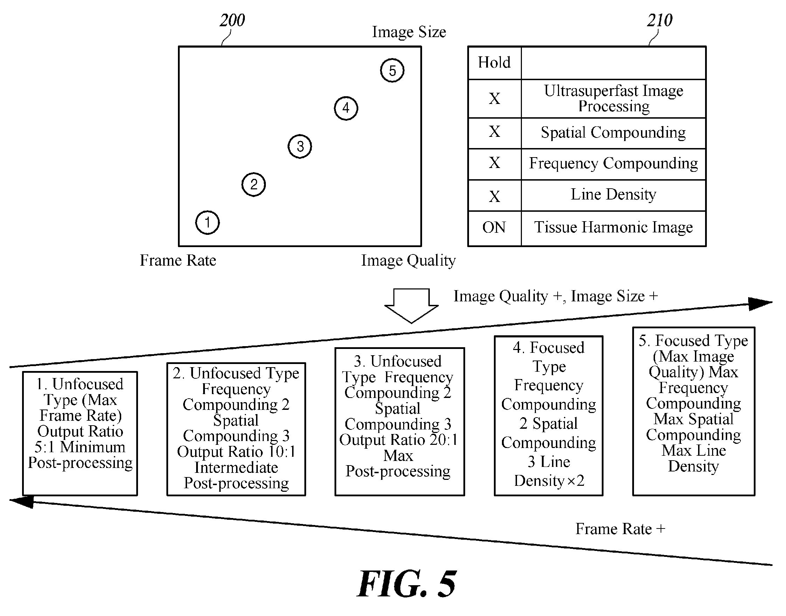

As for the of selecting the focused ultrasound or the unfocused ultrasound, upon determining the input position on the first interface 200 (or the fourth interface 230) to be in the unfocus area, the control unit 136 controls the transmission control unit 122 to select the unfocused ultrasound. For example, when determining the input position on the first interface 200 as being present in areas ① to ③ with reference to FIG. 5, the control unit 136 recognizes the input position of the input signal as an "unfocus area" to control the transmission control unit 122 to select the unfocused ultrasound. In addition, when determining the input position on the fourth interface 230 as being present in somewhere between a central region and the leftmost area with reference to FIG. 2B, the control unit 136 recognizes the input position as being in an "unfocus area" to control the transmission control unit 122 to select the unfocused ultrasound.

In addition, the control unit 136 controls the transmission control unit 122 to select the focused ultrasound when the input position of the input signal is determined as being in the focus area on the first interface 200 (or the fourth interface 230). For example, when determining the input position of the input signal as being present in areas ③ to ⑤ on the first interface 200 with reference to FIG. 5, the control unit 136 recognizes the input position of the input signal as the "focus area" to control the transmission control unit 122 to select the focused ultrasound. In addition, when determining the input position of the input signal as being present in somewhere between the central area and the rightmost area on the fourth interface 230 with reference to FIG. 2B, the control unit 136 recognizes the input position of the input signal as being in the "focus area" to control the transmission control unit 122 to select the focused ultrasound.

The control unit 136 causes a second interface 210 to be displayed including a parameter section apart from the first interface 200. The control unit 136 applies, to the ultrasound image, a parameter which is set to "hold" on the second interface 210, wherein the parameter set to "hold" has a fixed value regardless of the input position on the first interface 200. According to another aspect of the present disclosure, the control unit 136 may have the ultrasound image constantly applied with the value of the parameter set to "hold" on the second interface 210 as a requisite parameter of the ultrasound image regardless of the input signal.

In addition, the control unit 136 causes an operational range for each parameter to be displayed on the parameter section included in the second interface 210, and follows the user instruction by the user input unit 138 to cause a threshold value of the parameter to be adjusted within the operational range. For example, the "ON" or "OFF" state of the hold can be determined for each of the parameters, and the operational range of each of the parameters can be selected between a minimum threshold value to a maximum threshold value. More specifically, when a minimum frame rate is set to be "30 frames," the operational range may be reset with reference to "minimum 30 frames" for each the image parameters included in the second interface 210.

The control unit 136 checks whether the input position on the first interface 200 is determined by the user instruction of the user input unit 138, and combines some of the plurality of image parameters based on the input position on the first interface 200. Here, the image parameters include at least one parameter of an ultrasuperfast parameter, a frequency compounding (FRCD) parameter, a spatial compounding (SPCD) parameter, a line density parameter, a tissue harmonic imaging parameter, a synthetic aperture parameter, a multi-beam parameter and a number-of-focal-point parameter. In addition, the image parameter further includes at least one of a display ratio parameter classified according to a display ratio, an aberration correction parameter and a dynamic transmission focusing parameter classified. The display ratio of the image parameter can be adjusted by an aberration correction or a dynamic transmission focusing process.

The following will describe a case where the frame-rate related parameters and the image-quality related parameters are exclusively included as requisite in the plurality of image parameters. The control unit 136 causes the first interface 200 to be displayed including the frame-rate related parameters and the image-quality related parameters. The control unit 136 applies, to the ultrasound image, the image parameters that gradually improve the frame rate while lowering the image quality in response to the input position advancing in a first direction from a first side of the first interface 200 to a second side. To put it in another way by an orthogonal coordinate plane with an x-axis and a y-axis intersecting centrally of the first interface 200, the control unit 136 may apply, to the ultrasound image, the image parameters that gradually improve the frame rate while decreasing the image quality in response to the input position advancing from the second and third quadrants of the first interface 200 to the first and fourth quadrants. In addition, the control unit 136 applies, to the ultrasound image, the image parameters that gradually improve the image quality while lowering the frame rate in response to the input position advancing from the second side to the first side of the first interface 200 inclusive of the frame- rate related parameters and the image-quality related parameters. For example, the control unit 136 may apply, to the ultrasound image, the image parameters that gradually improve the image quality while decreasing the frame rate in response to the input position advancing from the first and fourth quadrants to the second and third quadrants of the orthogonal coordinate plane on the first interface 200.

In the operation of the control unit 136 related to adjustment of the image-quality related parameters, when the input position is moved to an area in excess of a threshold value on the first interface 200, the control unit 136 has the ultrasound image applied with the image parameters of the focused mode (for example, cancels the setting of parameters for an ultrasuperfast image processing) among the plurality of image parameters. In addition, when the input position is moved to the area within the preset threshold value on the first interface 200, the control unit 136 has the ultrasound image applied with the image parameters of the unfocused mode (for example, parameters for an ultrasuperfast image processing) among the plurality of image parameters. Here, the parameter for the ultrasuperfast image processing is adapted to cause the transducer 110 to transmit the unfocused ultrasound to the subject.

Where the image-size related parameters are included as optional in the first interface 200, the control unit 136 causes the image-size related parameters to additionally appear on the first interface 200 along with the frame-rate related parameters and the image-quality related parameters. The control unit 136 causes an image size of ultrasound image to be increased as the input position is moved from one side to the other end on the first interface 200 including the image-size related parameters. For example, the control unit 136 may increase the image size of the ultrasound image as the input position is moved from the third and fourth quadrants to the first and second quadrants on the orthogonal coordinates plane of the first interface 200 including the image-size related parameters. In addition, the control unit 136 reduces the image size of the ultrasound image as the input position is moved from the other end to the one end on the first interface 200 including the image size parameter. For example, the control unit 136 can reduce the image size of the ultrasound image as the input position is moved from first and second quadrants to the third and fourth quadrants on the orthogonal coordinate plane of the first interface 200 including the image-size related parameters.

In case where the image-size related parameters is implemented by the third interface 220, the control unit 136 causes the third interface 220 to appear to adjust the image size besides the frame-rate related parameters and the image-quality related parameters. The control unit 136 increases the image size of the ultrasound image in response to the input position advancing in a first direction from a first side to a second side on the third interface 220. In addition, the control unit 136 reduces the image size of the ultrasound image in response to the input position advancing from the second side to the first side on the third interface 220.

The scan converting unit 139 causes a scanning direction of data obtained by the signal processing unit 134 to be aligned with a pixel direction of a display unit (for example, a monitor), and performs mapping of the corresponding data to a pixel position of the display unit. The scan converting unit 139 converts the ultrasound image data into a data type used in a scan line type display unit.

The user input unit 138 receives an instruction by operation or input of a user. Here, the user instruction is for adjusting the image parameter of the ultrasound image, and a concept including a click, a drag, a zoom, a scroll, a press, and so on. The user input unit 138 according to some embodiments receives an input signal for simultaneously adjusting the plurality of image parameters (at least two parameters) of the ultrasound image. The user input unit 138 may be implemented by at least one input means of a touch panel, a knob and a slide bar among others.

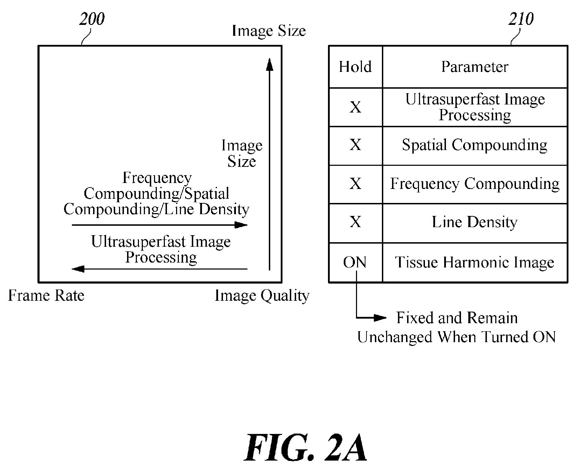

FIG. 2A is a view showing adjustments of the ultrasound image setting according to the embodiment.

The ultrasound medical apparatus 100 according to some embodiments can adjust the setting items of frame rate, image quality, image size, and so on, using the first interface 200 configured to combine setting items of the ultrasound image. Here, the setting items that can be adjusted by the ultrasound medical apparatus 100 are not limited to the frame rate, the image quality and the image size.

The ultrasound medical apparatus 100 may be implemented such that the second interface 210 is separately shown from the first interface 200 and a hold function of minimizing an abrupt image variation to the second interface 210 is added to fix the image parameter set by the user not to change the corresponding image processing result. Accordingly, the user can obtain the desired ultrasound image through a single input with no necessity of distinguishing complex image parameters for the ultrasound image setting.

The image parameters included in the second interface 210 includes an ultrasuperfast image processing parameter for determining whether an unfocused ultrasound is used for the ultrasuperfast image processing, a frequency compounding parameter for compounding images using different frequencies, a spatial compounding parameter for compounding reflected signals of different spaces, a line density parameter for setting a density of a scanned image, a tissue harmonic imaging parameter for generating an image using a harmonic wave ingredient, a synthetic aperture parameter, a multi-beam parameter for determining a plurality of beams are transmitted, a number of focal-point parameter for setting a focusing position, and so on. The image parameters included in the second interface 210 may further include a display ratio parameter for determining an output ratio, an aberration correction parameter for determining a chromatic aberration correction, a dynamic transmission focusing parameter, and so on.

In FIG. 2A, when the user operates the user input unit 138 to adjust the input position of the input signal on the first interface 200 in an image quality parameter direction (an arrow "→" direction of FIG. 2A), the "line density parameter," the "synthetic aperture parameter," the "frequency compounding parameter" and the "spatial compounding parameter" are activated (applied to ON), and the corresponding value is increased to improve the image quality according to an adjustment position on the first interface 200. Such image parameters have values that are gradually varied according to the adjustment positions on the first interface 200.

In addition, when the user operates the user input unit 138 to input the input position on the first interface 200 in a frame rate parameter direction (an arrow "←" direction of FIG. 2A), values of the "line density parameter," the "synthetic aperture parameter" and the "frequency compounding parameter" are reduced or inactivated (applied to "OFF") to increase the frame rate. In addition, when the user operates the user input unit 138 to input the input position on the first interface 200 in the frame rate parameter direction (the "←" direction of FIG. 2A), the transducer 110 transmits the unfocused ultrasound to the subject. Meanwhile, when the user operates the user input unit 138 to input the input position on the first interface 200 in the image quality parameter direction ("→" of FIG. 2A), the transducer 110 transmits the focused mode of ultrasound to the subject. In addition, the frame rate or the image quality can be adjusted by only the unfocused mode (due to transmission of the unfocused ultrasound) or the frame rate or the image quality can be adjusted by only the focused mode according to options of the image parameters included in the second interface 210.

In addition, when the user operates the user input unit 138 to input the input position on the first interface 200 in an image size increase direction (an arrow "↑" direction of FIG. 2A), the image size is increased. Meanwhile, when the user operates the user input unit 138 to input the input position on the first interface 200 in an image size decrease direction (an arrow "↓" direction of FIG. 2A), the image size is reduced.

As for the second interface 210 with reference to FIG. 2A, when the "hold" is set (selected to "ON" from "ON" and "OFF") on the second interface 210, the corresponding function is fixed to a current state and can be fixed in an image state desired by the user. In other words, when a "tissue harmonic imaging parameter (THI)" for generating the image using the harmonic wave ingredient among the plurality of image parameters included in the second interface 210 is set to "hold" (set to "ON"), the "tissue harmonic imaging parameter (THI)" has a currently fixed value (fixed to "ON") reflected to the ultrasound image processing. The "hold" that is a condition setting concept is not set only in the second interface 210 but, for example, an option of "minimum 30 frames," a "focused mode," an "unfocused mode," and so on, can be set to "hold." In addition, the plurality of image parameters included in the second interface 210 may be set according to a preset priority, and may be divided into parameters that have operational ranges and parameters that do not have operational ranges according to the image parameters.

FIG. 2B is a view showing adjustments of an ultrasound image setting according to another embodiment.

The user can adjust the image size by using the third interface 220 shown in FIG. 2B or adjust the frame rate and the image quality by using the fourth interface 230. The fourth interface 230 of FIG. 2B may be implemented by including only the "frame-rate related parameters" and the "image-quality related parameters" and the "image-size related parameters" may be implemented by the separate third interface 220.

When the user operates the user input unit 138 to input the input position on the third interface 220 in an "image size +" direction ("→" of FIG. 2B), the image size is increased. Meanwhile, when the user operates the user input unit 138 to input the input position on the third interface 220 in an "image size -" direction ("←" of FIG. 2B), the image size is reduced. Description of the fourth interface 230 including only the "frame-rate related parameter" and the "image-quality related parameters" is similar to those of FIG. 2B, and thus, description thereof will be omitted in FIG. 2B.

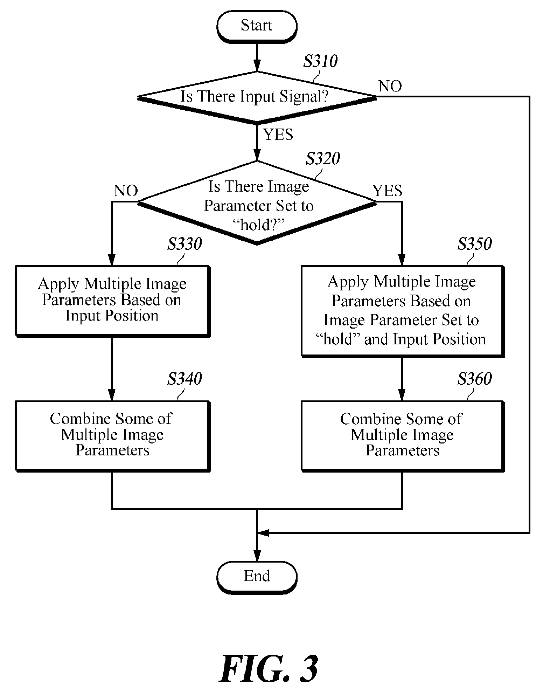

FIG. 3 is a flowchart for describing a method for adjusting an ultrasound image setting according to some embodiments.

The ultrasound medical apparatus 100 checks whether the input position input onto the first interface 200 is determined (S310).

As a result of checking in Step S310, when the input position is determined on the first interface 200, the ultrasound medical apparatus 100 generates the input signal corresponding to the input position, causes the second interface 210 including the parameter items to be shown separately from the second interface 210, and checks whether a parameter set to the "hold" on the second interface 210 is present (S320). In Step S320, the ultrasound medical apparatus 100 can cause an operational range to be displayed according to the parameters included in the second interface 210, and cause a threshold value of the parameter to be adjusted within the operational range.

As a result of checking in Step S320, when there is no parameter set to the "hold" among the parameters, the ultrasound medical apparatus 100 applies the plurality of image parameters based on the input position on the first interface 200 (S330). Here, the plurality of image parameters includes the "frame-rate related parameters" and the "image-quality related parameters" as requisite, and the "frame-rate related parameters" and the "image-quality related parameters" have a trade-off relation.

In Step S330, the ultrasound medical apparatus 100 arranges the frame-rate related parameters and the image-quality related parameters to be exclusively included as requisite in the first interface 200, and applies, to the ultrasound image, the image parameters that gradually improve the frame rate while lowering the image quality in response to the input position advancing in a first direction from a first side of the first interface 200 to a second side. In addition, the ultrasound medical apparatus 100 applies, to the ultrasound image, the image parameters that gradually improve the image quality while lowering the frame rate in response to the input position advancing from the second side of the first interface 200 to the first side. In other words, the frame rate and the image quality are simultaneously adjusted by a single input.

In addition, the ultrasound medical apparatus 100 applies the focused mode of image parameter among the plurality of image parameters to the ultrasound image when the input position on the first interface 200 is moved to the area of a preset threshold value or more. The ultrasound medical apparatus 100 causes the focused ultrasound to be selected when the focused mode of image parameter is applied. Meanwhile, the ultrasound medical apparatus 100 applies the unfocused mode of image parameter (for example, the ultrasuperfast image processing parameter) among the plurality of image parameters to the ultrasound image when the input position on the first interface 200 is moved to the area of the preset threshold value or less. The ultrasound medical apparatus 100 causes the unfocused ultrasound to be selected when the unfocused mode of image parameter is applied. In other words, the ultrasound medical apparatus 100 causes some of the plurality of image parameters to be applied according to the input signal with no complicate classification of the image parameters in Step S330.

In addition, the ultrasound medical apparatus 100 may further include the image-size related parameters on the first interface 200, and increase the image size of the ultrasound image as the input position on the first interface 200 is moved from the one end to the other end or decrease the image size of the ultrasound image as the input position on the first interface 200 is moved from the other end to the one end. Here, the "image-size related parameters" may be selectively applied as a separate concept from the "frame-rate related parameters" and the "image-quality related parameters."

After Step S330, the ultrasound medical apparatus 100 combines some of the plurality of image parameters based on the input position on the first interface 200 (S340).

Meanwhile, when there is a parameter set to the "hold" among the parameters in Step S320, the ultrasound medical apparatus 100 applies the plurality of image parameters based on the parameter set to the "hold" and the input signal (S350). In step S350, the ultrasound medical apparatus 100 applies, to the ultrasound image, the parameter which is set to "hold" on the second interface 210 and has a fixed value regardless of the input position on the first interface 200. The ultrasound medical apparatus 100 combines some of the plurality of image parameters based on the parameter set to the "hold" and the input signal (S360). For example, when the "line density parameter" having a value of "x2" on the second interface 210 is set to the "hold" in Step S360, the ultrasound medical apparatus 100 can apply the "line density parameter" having the value of "x2" to the ultrasound image as a value (for example. "x2") fixed regardless of the input position on the first interface 200 or a variation in the input position.

Although Steps S310 to S360 are described to be sequentially performed in the example shown in FIG. 3, it merely instantiates a technical idea of some embodiments of the present disclosure. Therefore, a person having ordinary skill in the pertinent art could appreciate that various modifications, additions, and substitutions are possible by changing the sequences described in FIG. 3 or by executing two or more steps in parallel, without departing from the gist and the nature of the embodiments of the present disclosure, and hence FIG. 3 is not limited to the illustrated chronological sequence.

As described above, the method of adjusting the ultrasound image setting according to the embodiment shown in FIG. 3 may be recorded on a computer-readable recording medium that can be executed by a program. The computer-readable recording medium on which the program configured to execute the method of adjusting the ultrasound image setting according to the embodiment is recorded includes all kinds of recording media on which computer-readable data are stored.

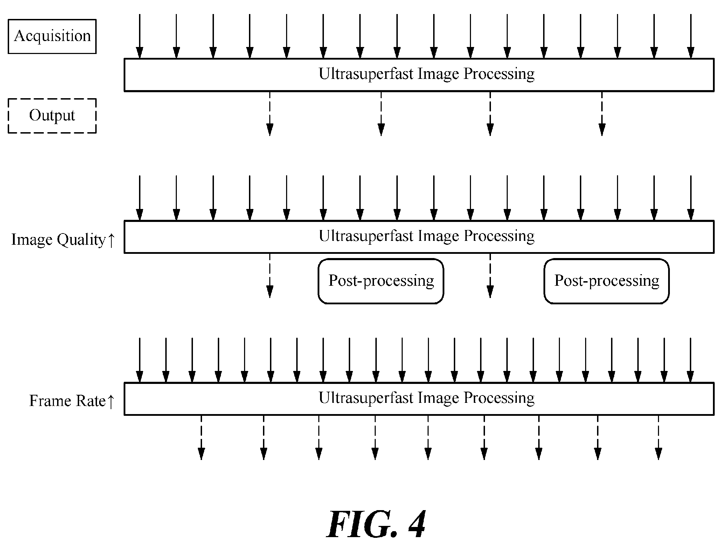

FIG. 4 is a view showing adjustment of an output ratio according to some embodiments.

When the image quality is improved while the ultrasuperfast image processing is performed by the ultrasound medical apparatus 100 according to some embodiments, the image parameter is applied to perform a larger amount of post-processing for a corresponding time by reducing an output period. On the contrary, the ultrasound medical apparatus 100 applies the image parameter as the number of data acquisitions and the output period are increased to reduce the post-processing when the frame rate is increased.

More specifically, in the post-processing shown in FIG. 4, the post-processing is "data analysis" or "data processing." First, the "data analysis" serving as the post-processing is referred to as a process of referring comparison result of phase differences and so on of data currently obtained by the ultrasound medical apparatus 100 and data obtained after that upon the following ultrasound (the focused ultrasound or the unfocused ultrasound) transmission (Tx). In addition, the "data processing" serving as the post-processing is referred to as a process of allowing the ultrasound medical apparatus 100 to perform processing of data using a preset parameter. For example, the ultrasound medical apparatus 100 can apply the dynamic focusing using the preset parameter upon the ultrasound (the focused ultrasound or the unfocused ultrasound) transmission (Tx).

FIG. 5 is a diagram of a method for combining some of a plurality of image parameters according to some embodiments of the present disclosure.

The ultrasound medical apparatus 100 applies the image parameter corresponding to a position □ to the ultrasound image when the input position on the first interface 200 is the position □ according to the user instruction of the user input unit 138 with reference to FIG. 5. For example, the "ultrasuperfast image processing parameter" may be applied to become unfocused mode (a maximum frame rate) among the image parameter, the "output ratio parameter" having a ratio of 5:1 may be applied, and a "minimum post-processing" may be applied.

The ultrasound medical apparatus 100 applies the image parameter corresponding to the position ② to the ultrasound image when the input position on the first interface 200 is disposed at a position ② (or moved from the position ① to the position ②) according to the user instruction of the user input unit 138 with reference to FIG. 5. For example, the "ultrasuperfast image processing parameter" may be applied to become the unfocused mode of parameter among the image parameters, the "frequency compounding parameter" using two frequencies may be applied, the "spatial compounding parameter" of compounding three spaces may be applied, the "output ratio parameter" having a ratio of 10:1 may be applied, and an "intermediate post-processing" ratio may be applied.

The ultrasound medical apparatus 100 applies the image parameter corresponding to a position ③ to the ultrasound image when the input position on the first interface 200 is disposed at the position ③ (or moved from the position ② to the position ③) according to the user instruction of the user input unit 138 with reference to FIG. 5. For example, the "ultrasuperfast image processing parameter" may be applied to become the unfocused mode of parameter among the image parameters, the "frequency compounding parameter" using two frequencies may be applied, the "spatial compounding parameter" of compounding three spaces may be applied, the "output ratio parameter" having a ratio of 20:1 may be applied, and a "maximum post-processing" ratio may be applied.

The ultrasound medical apparatus 100 applies the image parameter corresponding to a position ④ to the ultrasound image when the input position on the first interface 200 is disposed at the position ④ (or moved from the position ③ to the position ④) according to the user instruction of the user input unit 138 with reference to FIG. 5. For example, the "ultrasuperfast image processing parameter" may be released to become the focused mode of parameter among the image parameters, the "frequency compounding parameter" using two frequencies may be applied, the "spatial compounding parameter" of compounding three spaces may be applied, and the "line density parameter" having a value of "2x" may be applied.

The ultrasound medical apparatus 100 applies the image parameter corresponding to a position ⑤ to the ultrasound image when the input position on the first interface 200 is disposed at the position ⑤ (or moved from the position ④ to the position ⑤) according to the user instruction of the user input unit 138 with reference to FIG. 5. For example, the "ultrasuperfast image processing parameter" may be released to become the focused mode of parameter among the image parameters, the "frequency compounding parameter" having a "maximum" value may be applied, the "spatial compounding parameter" having a "maximum" value may be applied, and the "line density parameter" having a "maximum" value may be applied.

The ultrasound medical apparatus 100 causes the unfocused ultrasound to be selected when the input position on the first interface 200 is in areas (unfocus areas) of □ to ③according to the user instruction of the user input unit 138 with reference to FIG. 5. Meanwhile, the ultrasound medical apparatus 100 may cause the focused ultrasound to be selected when the input position of the input signal input onto the first interface 200 is in areas (focus areas) of ③to ⑤ according to the user instruction of the user input unit 138 with reference to FIG. 5. Here, the ultrasound medical apparatus 100 may apply a value of the parameter set to the "hold" on the second interface 210 to the ultrasound image as a value fixed regardless of "any one area among the areas □ to ⑤" or "a variation in the areas □ to ⑤" serving as the input position of the input signal input onto the first interface 200.

Although exemplary embodiments of the present disclosure have been described for illustrative purposes, those skilled in the art will appreciate that various modifications, additions and substitutions are possible, without departing from the idea and scope of the claimed invention. Specific terms used in this disclosure and drawings are used for illustrative purposes and not to be considered as limitations of the present disclosure. Therefore, exemplary embodiments of the present disclosure have been described for the sake of brevity and clarity. Accordingly, one of ordinary skill would understand the scope of the claimed invention is not to be limited by the explicitly described above embodiments but by the claims and equivalents thereof.

### Reference Sign List

100: ultrasound medical apparatus 110: transducer
120: front end 130: host

### CROSS-REFERENCE TO RELATED APPLICATION

This application claims priority under 35 U.S.C §119(a) of Patent Application No. 10-2013-0152326, filed on December 9, 2013 in Korea, the entire content of which is incorporated herein by reference. In addition, this non-provisional application claims priority in countries, other than the U.S., with the same reason based on the Korean patent application, the entire content of which is hereby incorporated by reference.

## Claims

1. An ultrasound medical apparatus, comprising:
a user input unit configured to receive a user instruction;
a transmission control unit configured to operate elements of a transducer for outputting a focused ultrasound or an unfocused ultrasound; and
a control unit configured to be responsive to an input position determined on a first interface by the user instruction, for generating an input signal corresponding to the input position and causing one of the focused ultrasound and the unfocused ultrasound to be selected based on the input signal.

2. The ultrasound medical apparatus of claim 1, wherein the control unit is configured to combine some of a plurality of image parameters based on the input position and to apply combined image parameters to an ultrasound image.

3. The ultrasound medical apparatus of claim 2, wherein
the first interface is configured to comprise a focus area and an unfocus area for adjusting the ultrasound image, and
the control unit is configured to
combine focusing parameters based on the input position in the focus area to apply combined focusing parameters to the ultrasound image, or
combine unfocusing parameters based on the input position in the unfocus area to apply combined unfocusing parameters to the ultrasound image.

4. The ultrasound medical apparatus of claim 2, wherein the control unit is configured to
display a second interface comprising an itemized listing of parameters separately from the first interface, and
apply, to the ultrasound image, at least one parameter which is set to "hold" on the second interface, wherein the at least one parameter set to "hold" has a fixed value regardless of the input position on the first interface.

5. The ultrasound medical apparatus of claim 4, wherein the control unit is configured to
cause an operational range to be displayed for each of parameters in the itemized listing, and
cause a threshold value of each parameter to be adjusted within the operational range based on the user instruction.

6. The ultrasound medical apparatus of claim 2, wherein the control unit is configured to
apply, to the ultrasound image, the image parameters that gradually improve a frame rate while lowering an image quality in response to the input position advancing in a first direction from a first side of the first interface to a second side, and
apply, to the ultrasound image, the image parameters that gradually improve the image quality while lowering the frame rate in response to the input position advancing from the second side of the first interface to the first side.

7. The ultrasound medical apparatus of claim 2, wherein the control unit is configured to
increase an image size of the ultrasound image as the input position is moved from a first side of the first interface to a second side, and
reduce the image size of the ultrasound image as the input position is moved from the second side of the first interface to the first side.

8. The ultrasound medical apparatus of claim 2, wherein the image parameters comprise:
at least one parameter of an ultrasuperfast image processing parameter, a frequency compounding (FRCD) parameter, a spatial compounding (SPCD) parameter, a line density parameter, a tissue harmonic imaging (THI) parameter, a synthetic aperture parameter, a multi-beam parameter, a number-of-focal-point parameter, a display ratio parameter, an aberration correction parameter and a dynamic transmission focusing parameter.

9. The ultrasound medical apparatus of claim 1, wherein the user input unit comprises:
at least one input means of a touch panel, a knob and a slide bar.

10. A method for adjusting an ultrasound image setting, the method comprising:
detecting whether an input position is determined on a first interface by a user instruction;
generating an input signal corresponding to the determined input position; and
controlling to cause one of a focused ultrasound and an unfocused ultrasound to be selected based on the input signal.

11. The method of claim 10, wherein the controlling comprises:
combining some of a plurality of image parameters based on the input position and applying combined image parameters to the ultrasound image.

12. The method of claim 11, wherein the controlling comprises:
combining focusing parameters based on the input position in a focus area of the first interface to apply combined focusing parameters to the ultrasound image, or
combining unfocusing parameters based on the input position in an unfocus area of the first interface to apply combined unfocusing parameters to the ultrasound image.

13. The method of claim 11, wherein the controlling comprises:
applying, to the ultrasound image, at least one parameter which is set to "hold" on a second interface comprising the itemized listing of parameters, wherein the at least one parameter set to "hold" has a fixed value regardless of the input position.
